# EUROPÄISCHE PATENTSCHRIFT

(11) **EP 3 259 102 B1**
(45) Veröffentlichungstag und Bekanntmachung des Hinweises auf die Patenterteilung: **08.07.2020**
(21) Anmeldenummer: 16717536.3
(22) Anmeldetag: 19.02.2016
(51) Int. Cl.: B26D 3/16, B26D 7/18

(54) **ANLAGE SOWIE VERFAHREN ZUM HERSTELLEN EINES BEUTELS FÜR MEDIZINISCHE ZWECKE**
SYSTEM AND METHOD FOR PRODUCING A BAG FOR MEDICAL PURPOSES
SYSTÈME ET PROCÉDÉ DE FABRICATION D'UN SAC À DES FINS MÉDICALES

(30) Priorität: 20.02.2015 DE 102015002006
(43) Veröffentlichungstag der Anmeldung: 27.12.2017
(73) Patentinhaber: Kiefel GmbH, 83395 Freilassing (DE)
(72) Erfinder: STEIN, Bernd, 83417 Kirchanschöring (DE); GSCHWENDTNER, Rupert, 83435 Bad Reichenhall (DE)
(74) Vertreter: FARAGO Patentanwälte
(86) Internationale Anmeldenummer: PCT/DE2016/000061
(87) Internationale Veröffentlichungsnummer: WO 2016/131440

(56) Entgegenhaltungen:
- EP-A2- 1 500 451
- BE-A1- 883 828
- US-A- 3 214 312
- US-A- 3 432 986

## Beschreibung

Die Erfindung betrifft einerseits eine Anlage zum Herstellen eines Beutels für medizinische Zwecke umfassend eine Schlauchübergabestation mit einem Übergabemittel zum Übergeben eines der Schlauchübergabestation zulaufenden Schlauchs an einen Halter und zum dortigen Abtrennen eines an den Halter übergebenen Schlauchstücks von einem Restschlauch des zulaufenden Schlauchs, um anschließend das abgetrennte Schlauchstück mit zwei Folienlagen zum Bilden eines Zugangs an den Beutel verbindbar machen zu können.

Die Erfindung betrifft andererseits ein Verfahren zum Herstellen eines Beutels für medizinische Zwecke, bei welchem ein einer Schlauchübergabestation zulaufender Schlauch mittels eines Übergabemittels an einen Halter zum dortigen Abtrennen eines an den Halter übergebenen Schlauchstücks von einem Restschlauch des zulaufenden Schlauchs übergeben wird, so dass das Schlauchstück anschließend mit zwei Folienlagen zum Bilden eines Zugangs an dem Beutel verbunden wird.

Gattungsgemäße Anlagen und Verfahren, um ein einzelnes Schlauchstück, welches später Bestandteil eines Zugangs eines medizinischen Beutels ist, von einem Schlauch abzutrennen, sind aus dem Stand der Technik hinlänglich bekannt. Insbesondere ist bekannt, ein Schlauch zu einer Schlauchaufsteck- bzw. Schlauchübergabestation zu führen und dort in einzelne Schlauchstücke zu trennen.

Dabei werden einzelne Schlauchstücke von einem "Endlosschlauch" abgetrennt, um sie nachher vereinzelt an den Folien bereitstellen zu können. Hierzu wird ein Ende dieses Endlosschlauchs einem Halter zugeführt und dort fixiert, so dass ein dann von einem Restschlauch des Endlosschlauchs abgetrenntes Schlauchstück durch den Halter gehalten ist. Die so abgetrennten Schlauchstücke werden nach ihrem Abtrennen mittels einer entsprechend ausgebildeten Apparatur zwischen zwei Folien gelegt und anschließend mit den zwei Folien zu einem medizinischen Beutel verschweißt.

Es versteht sich, dass bereits bei der Herstellung derartiger medizinischer Beutel zwingend eine Verunreinigung durch Schmutz-, Material-, prozessbedingten Partikeln oder ähnlichem zu vermeiden ist, um die Gefahr einer Kontamination des später in dem medizinischen Beutel bevorrateten medizinischen Produkts zu reduzieren bzw. zur Gänze auszuschließen.

So offenbart die internationale Offenlegungsschrift WO 2014 /154 195 A1 eine Anlage und ein Verfahren zur Herstellung von Infusionsbeuteln aus zwei Folienlagen mit einem Zugangsmittel und einem Injektionsstopfen. Der Zugang weist einen Schlauch aufweist, wobei ein Injektionsstopfen mit daran einstückig ausgebildetem Zugang zwischen die Folienlagen eingeschweißt wird. Bei dem hier vorgeschlagenen System ist die Öffnung also direkt in den Beutel eingeschweißt. Der Schlauch entfällt somit als Zwischenstück. Darüber hinaus können das flexible Rohr und der Anschluss optional mit demselben Schweißwerkzeug eingeschweißt werden. Soll ein flexibler Schlauch und ein Anschluss oder zwei flexible Schläuche eingeschweißt werden, müssen keine Werkzeuge gewechselt werden.

Aus dem Stand der Technik ist durch die US 3 214 312 A eine Vorrichtung zum Herstellen eines Sackes bekannt, welche Rollen zum Übergeben eines zulaufenden Schlauches an eine Formeinrichtung umfasst, wobei durch eine Öffnung Luft in den zulaufenden Schlauch eingeblasen wird, so dass sich der Schlauch innerhalb der Formeinrichtung aufweitet und dabei mit einem erhitzten ringförmigen Schneidmittel in Kontakt kommt, so dass der Schlauch durch diese Schneidmittel durchgetrennt wird.

In der BE 838 828 A1 ist eine Maschine zum Herstellen von Kunststoffbeuteln beschrieben. Die Maschine hat Antriebsrollen zum Zuführen eines Bandes, ein Messer zum Trennen des Bandes an einer Trennstelle sowie eine Blaseinrichtung mit Düsen, welche auf die Trennstelle gerichtet sind, wobei die Düsen zum Kühlen einer Schweißschiene bzw. einer hierdurch entstandenen Schweißung am Beutel dienen.

Ferner offenbart die US 3 432 986 A eine Verpackungsmaschine, mittels welcher Behälter geformt, gefüllt und geschlossen werden können. Zum Formen der Behälter wird Pressluft mit niedrigen Druck in den Schlauch eingebracht, um ein Kollabieren des Schlauchs zu vermeiden.

Die EP 1 500 451 A2 zeigt ein Verfahren sowie eine Vorrichtung zum Entgraten von Rohren. Die Vorrichtung verfügt über ein Entgratungswerkzeug, welches einem Schneidwerkzeug gegenüberliegend angeordnet ist. Zum Kühlen der Schnittflächen und zum Befreien der Trennstelle von Spänen ist für ein Luftstrom gesorgt.

Der Erfindung liegt die Aufgabe zugrunde, insbesondere gattungsgemäße Anlagen zum Herstellen eines Beutels für medizinische Zwecke derart weiterzuentwickeln, dass die Gefahr von Verunreinigungen insbesondere an einem durch einen Schlauch formulierten Zugangs des Beutels reduziert ist.

Die Aufgabe der Erfindung wird von einer Anlage zum Herstellen eines Beutels für medizinische Zwecke umfassend eine Schlauchübergabestation mit einem Übergabemittel zum Übergeben eines der Schlauchübergabestation zulaufenden Schlauchs an einen Halter und zum dortigen Abtrennen eines an den Halter übergebenen Schlauchstücks von einem Restschlauch des zulaufenden Schlauchs gelöst, um zum Bilden eines Zugangs an den Beutel anschließend das abgetrennte Schlauchstück mit zwei Folienlagen zu dem Beutel verbindbar machen zu können, wobei die Schlauchübergabestation wenigstens eine Luftdüse umfasst, und wobei ein Wirkbereich der wenigstens einen Luftdüse auf eine designierte Stelle zum Abtrennen gerichtet ist, so dass eine durch ausgeblasene Luft und/oder eingesaugte Luft erzwungene Luftströmung an der designierten Stelle zum Abtrennen einstellbar ist, um beim Abtrennen des Schlauchstücks von dem zulaufenden Schlauch erzeugte Schlauchmaterialrestpartikel von der designierten Stelle bzw. von dem Schlauchstück und auch noch von dem Restschlauch durch die erzwungene Luftströmung zu entfernen.

Dadurch, dass die Schlauchübergabestation wenigstens mit einer derartigen Luftdüse ausgerüstet ist, können beim Abtrennen des Schlauchstücks von dem zulaufenden Schlauch gegebenenfalls erzeugte Schlauchmaterialrestpartikel von der Trennstelle (designierte Stelle) bzw. von dem Schlauchstück und auch noch von dem Restschlauch des zulaufenden Schlauchs durch die erzeugte Luftströmung entfernt werden.

Hierdurch wird die Gefahr einer Kontaminierung des Schlauchstücks und damit auch des späteren Zugangs des medizinischen Beutels sowie dessen Inhalt durch Schlauchmaterialrestpartikel signifikant reduziert, wenn nicht sogar zur Gänze ausgeschlossen.

Vorteilhaft ist es ferner, wenn die Schlauchübergabestation eine Luftionisiereinrichtung umfasst, um im Wesentlichen das abgetrennte Schlauchstück und gegebenenfalls auch noch den Restschlauch direkt an der Schlauchübergabestation zu sterilisieren.

Bevorzugt ist diese Luftionisiereinrichtung konstruktiv der wenigstens einen Luftdüse zugeordnet, so dass die aus dieser Luftdüse ausströmende Luft ionisiert werden kann.

Zum Erzeugen einer erzwungenen Luftströmung kann die wenigstens eine Luftdüse mittels einer entsprechenden Schlaucheinrichtung an einem Druckdifferenzmittel zum Erzeugen eines Überdrucks und/oder eines Unterdrucks fluidisch angeschlossen sein.

Bevorzugt weist die wenigstens eine Luftdüse ein Druckdifferenzmittel zum Erzeugen eines Überdrucks und/oder eines Unterdrucks an der Luftdüse auf, so dass im Betrieb des Druckdifferenzmittels eine durch ausgeblasene Luft und/oder eingesaugte Luft erzwungene Luftströmung an der designierten Stelle zum Abtrennen einstellbar ist. Hierdurch kann auf eine zusätzliche Verrohrung verzichtet werden, wodurch eine diesbezügliche Anordnung noch kompakter realisiert werden kann. Ferner ist hierdurch selbst eine bereits bestehende Schlauchübergabestation leicht nachrüstbar.

Es versteht sich, dass die wenigstens eine Luftdüse derart ausgestaltet sein kann, dass mit ihr sowohl Luft ausgeblasen als auch Luft eingesaugt werden kann, beispielsweise indem die wenigstens eine Luftdüse zwei räumlich voneinander getrennte Luftkanäle aufweist.

Eine bevorzugte Ausführungsvariante sieht vor, dass zwei Luftdüsen vorgesehen sind, wovon eine erste Luftdüse zum Ausblasen von Luft und eine zweite Luftdüse zum Einsaugen von Luft eingerichtet ist. Zwar ist für diese Alternative mehr Bauraum erforderlich, jedoch kann hierdurch eine noch weiträumigere Trennung der zwei Luftkanäle erzielt werden.

Besonders vorteilhaft ist es, wenn die designierte Stelle zum Abtrennen zwischen den Luftdüsen angeordnet ist. Hierdurch kann mittels einer ersten Luftdüse der Luftdüsen Luft von einer ersten Seite her auf die Trenn- bzw. Schnittstelle geblasen werden, während mittels einer zweiten Luftdüse der Luftdüsen von einer anderen Seite her Luft von der Trenn- bzw. Schnittstelle abgesaugt wird.

Hierdurch gelingt es, einerseits gegebenenfalls vorhandene Schlauchmaterialrestpartikel von dem abgetrennten Schlauchstück und dem diesem Schlauchstück zugewandten Ende des zulaufenden Schlauchs fortzublasen und andererseits diese fortgeblasenen bzw. aufgewirbelten Schlauchmaterialrestpartikel sogleich abzusaugen, so dass auch die Umgebung der Schlauchübergabestation sogleich vor einer Kontamination durch umherschwirrende Schlauchmaterialrestpartikel geschützt ist.

Um die Schlauchübergabestation hinsichtlich Trenn- bzw. Schneidvorgänge variabler einstellen zu können, ist es vorteilhaft, wenn die wenigstens eine Luftdüse verlagerbar gegenüber insbesondere der designierten Stelle angeordnet ist.

Um die Schlauchmaterialrestpartikel im Wesentlichen unmittelbar an ihrem Entstehungsort von der Schlauchübergabestation entfernen bzw. absaugen zu können, ist es besonders vorteilhaft, wenn zumindest die zweite Luftdüse zum Einsaugen von Luft schwenkbar an der Schlauchübergabestation angeordnet ist.

Eine solche Schwenkbarkeit der zweiten Luftdüse kann an der Schlauchübergabestation konstruktiv einfach gewährleistet werden, wenn die zweite Luftdüse zum Einsaugen von Luft an einer schwenkbaren Halteeinrichtung zum Halten eines Trennmittels zum Trennen des zulaufenden Schlauchs angeordnet ist.

Die so ausgestaltete schwenkbare Halteeinrichtung kann beispielsweise in einem 3D-Druckverfahren hergestellt werden.

Eine derartige schwenkbare Halteeinrichtung ist nachfolgend beispielhaft noch im Zusammenhang mit einem Messer zum Trennen des zulaufenden Schlauchs erläutert, wobei das Messer schenkbar bzw. klappbar an einem Schneidkopf angeordnet ist.

Ferner kann die Entstehung von Schlauchmaterialrestpartikeln bereits dadurch signifikant reduziert werden oder sogar gänzlich vermieden werden, wenn die Schlauchübergabestation halterseits ein Schlauchzughaltemittel und andererseits ein Schlauchziehmittel aufweist, welche dazu eingerichtet sind, zwischen dem abgetrennten Schlauchstück und dem Restschlauch des zulaufenden Schlauchs Zugkräfte anzubringen, während das Abtrennen erfolgt.

Vorliegend ist die Schlauchübergabestation insbesondere derart ausgestaltet, dass ein an den Halter übergebenes und abzutrennendes Schlauchstück bereits vor dem Abtrennen zumindest temporär durch ein Schlauchzughaltemittel ortsfest an dem Halter fixiert wird.

Des Weiteren kann mittels der Schlauchübergabestation Zugkräfte in den so eingeklemmten beziehungsweise fixierten Schlauch eingebracht werden, so dass der zulaufende Schlauch zumindest in einem Schlauchbereich zwischen dem Übergabemittel und dem Halter vorgespannt an der Schlauchübergabestation bereitgestellt werden kann, wodurch wiederum das an dem Halter übergebene Schlauchstück von dem auf Seiten des Übergabemittels befindlichen Restschlauch des zulaufenden Schlauchs derart getrennt werden kann, dass durch den eigentlichen Trennvorgang signifikant weniger Schlauchmaterialrestpartikel in die Umgebung gelangen.

Mit anderen Worten bedeutet dies, dass während des Abtrennens weniger durch den Abtrennvorgang bedingte Schlauchmaterialrestpartikel erzeugt werden, so dass insgesamt hierdurch die Gefahr verringert ist, dass derartige Schlauchmaterialrestpartikel das abgetrennte Schlauchstück einerseits aber auch den Restschlauch des zulaufenden Schlauchs andererseits kontaminieren, wodurch die Gefahr verringert ist, dass derartige Schlauchmaterialrestpartikel letztendlich in das medizinische Produkt gelangen, welches in dem medizinischen Beutel bevorratet werden soll.

Kurz gesagt: Vorliegend ist es möglich, einen äußerst sauberen Schnitt an dem zulaufenden Schlauch durchzuführen.

Insofern wird die Aufgabe der Erfindung auch von einem Verfahren zum Herstellen eines Beutels für medizinische Zwecke gelöst, bei welchem ein einer Schlauchübergabestation zulaufender Schlauch mittels eines Übergabemittels an einen Halter zum dortigen Abtrennen eines an den Halter übergebenen Schlauchstücks von dem Restschlauch des zulaufenden Schlauchs übergeben wird, so dass zum Bilden eines Zugangs an dem Beutel das Schlauchstück anschließend mit zwei Folienlagen verbunden wird, wobei sich das Verfahren dadurch auszeichnet, dass insbesondere die folgenden Schritte durchgeführt werden, nämlich erstens Zuführen des zulaufenden Schlauchs zum Halter; zweitens zugfestes Halten des zulaufenden Schlauchs an dem abzutrennenden Schlauchstück auf Seiten des Halters mittels eines Schlauchzughaltemittels; drittens Längsziehen des zulaufenden Schlauchs zwischen dem Schlauchzughaltemittel und einer Zuführung, so dass eine Zugkräfte auf die designierte Stelle zum Abtrennen wirken; und viertens Abtrennen des Schlauchstücks von dem Restschlauch des zulaufenden Schlauchs, um beim Abtrennen des Schlauchstücks von dem zulaufenden Schlauch erzeugte Schlauchmaterialrestpartikel von der designierten Stelle bzw. von dem Schlauchstück und auch noch von dem Restschlauch durch die erzwungene Luftströmung zu entfernen.

Mittels dieses vorgeschlagenen Verfahrens ist es möglich, einen äußerst sauberen Schnitt an dem zulaufenden Schlauch durchzuführen, wodurch beim Durchtrennen signifikant weniger Schlauchmaterialrestpartikel erzeugt werden und in die Umgebung gelangen.

Hinsichtlich der vorliegenden Konstruktion kann der Halter in Abhängigkeit der Schlauchübergabestation, insbesondere des Übergabemittels, nahezu beliebig ausgestaltet sein.

Das dem Halter zugewandte Ende des zulaufenden Schlauchs beziehungsweise das abzutrennende Schlauchstück kann konstruktiv sehr einfach an den Halter übergeben werden, wenn der Halter einen Dorn aufweist, auf welchen der zulaufende Schlauch zumindest teilweise aufgesteckt werden kann.

Insofern kann hinsichtlich der Schlauchübergabestation auch von einer Schlauchaufsteckstation gesprochen werden, wobei an dieser Stelle ausdrücklich betont sei, dass kein "Aufstecken" erforderlich ist, um die Erfindung zu verwirklichen, sondern dass schon das reine Übergeben des Schlauchendes an einen Halter ausreichen kann.

Eine bevorzugte Ausführungsvariante sieht in diesem Zusammenhang vor, dass der Halter eine dornförmige Zentralelektrode aufweist, mittels welcher das Schlauchstück an einer weiteren Bearbeitungsstation der vorliegenden Anlage sogleich mit Folienlagen des Beutels verschweißt werden kann.

Insofern ist es verfahrenstechnisch auch zweckmäßig, wenn das Zuführen des zulaufenden Schlauchs auf einen Dorn erfolgt, insbesondere auf eine Zentralelektrode.

Gegebenenfalls kann mittels einer dornförmigen Zentralelektrode der zulaufende Schlauch sogleich an der Schlauchübergabestation erwärmt werden, da sich gezeigt hat, dass das an den Halter übergebene Schlauchstück aufgrund von besseren Gleiteigenschaften im erwärmten Zustand wesentlich besser von dem Restschlauch des zulaufenden Schlauchs getrennt werden kann.

Genauer gesagt, durch ein Erwärmen des zulaufenden Schlauchs in einem Bereich der Trennstelle kann die Bildung von durch einen Trennschnitt bedingten Schlauchmaterialrestpartikeln weiter reduziert werden.

Es hat sich gezeigt, dass das zu übergebene Schlauchstück einfach auf diesen Dorn aufgesteckt werden kann, wenn der Außendurchmesser des Dorns auf den Innendurchmesser des zulaufenden Schlauchs derart kompatibel angepasst sein muss, dass das abgetrennte Schlauchstück bevorzugt schon allein aufgrund einer Reibschlussverbindung an dem Dorn gehaltert werden kann, so dass das abgetrennte Schlauchstück betriebssicher von der Schlauchübergabestation zu einer weiteren Bearbeitungsstation der Anlage zum Herstellen eines Beutels transportiert werden kann.

Damit aber auch höhere Zugkräfte in den zulaufenden Schlauch eingebracht werden können, ohne die Gefahr, dass sich das abzutrennende Schlauchstück von dem Halter beziehungsweise von dem Dorn beziehungsweise von der dornförmigen Zentralelektrode löst, ist es vorteilhaft, wenn der Halter eine von außen angreifende Schlauchklemme aufweist.

Ist der Halter in Einbringrichtung des zulaufenden Schlauchs fixiert, kann der zulaufende Schlauch mittels der Übergabemittel betriebssicher auf einen entsprechend ortsfest fixierten Dorn beziehungsweise auf eine entsprechend ortsfest fixierte dornförmige Zentralelektrode aufgesteckt und Zugkräfte in den zulaufenden Schlauch eingeleitet werden, ohne dass der Halter sich in Einbringrichtung bzw. in Hauptwirkrichtung der Zugkräfte nachteilig bewegt.

Somit bleibt der Halter mit dem daran befestigten abzutrennenden Schlauchstück ortsfest positioniert, wenn an dem zulaufenden Schlauch zum Aufbringen beziehungsweise zum Einbringen der Zugkräfte entgegen der Einbringrichtung gezogen wird.

Insofern sieht eine entsprechend vorteilhaft ausgestaltete Verfahrensvariante auch vor, dass der zulaufende Schlauch an einem abzutrennenden Schlauchstück mit dem Halter gegen Zurückziehen fixiert wird, vor allem von außen festgeklemmt wird.

Alternativ oder kumulativ könnte eine Klemmung auch durch einen radial aufspreizfähigen Dorn oder durch eine radial aufspreizfähige dornförmige Zentralelektrode erfolgen.

Radial bedeutet vorliegend im Allgemeinen quer zur Einbringrichtung des zulaufenden Schlauchs.

Es versteht sich, dass auch das vorliegende Übergabemittel nahezu beliebig ausgestaltet sein kann.

Eine weitere bevorzugte Verfahrensvariante sieht vor, dass das Zuführen des Schlauchs durch einen Durchführkanal erfolgt, wodurch das dem Halter zugewandte Ende des zulaufenden Schlauchs genauer vor dem Dorn beziehungsweise vor der dornförmigen Zentralelektrode positioniert werden kann.

Um den zulaufenden Schlauch beim Übergeben an den Halter beziehungsweise beim Aufstecken auf den Dorn beziehungsweise auf die dornförmige Zentralelektrode in radialer Richtung zur Einbringrichtung, also seitlich, präziser führen zu können, ist es somit entsprechend vorteilhaft, wenn das Übergabemittel einen Durchführkanal für den zulaufenden Schlauch aufweist.

Der zulaufende Schlauch kann besonders präzise an den Halter übergeben werden, wenn das Übergabemittel in Einbringrichtung vor- und zurückfahrbar ist.

Mit anderen Worten bedeutet dies, dass speziell das Übergabemittel gegenüber dem Halter translatorisch in eine Schlauchzustellrichtung und in eine Schlauchrückstellrichtung verlagerbar in der Schlauchübergabestation gelagert ist.

Eine besonders bevorzugte Ausführungsvariante sieht hierbei vor, dass das Übergabemittel dazu eingerichtet ist, den Durchführkanal nah an den Halter zu verfahren, dann den zulaufenden Schlauch zumindest mit dem abzutrennenden Schlauchstück zu übergeben, und nach Abtrennen des Schlauchstücks den Restschlauch des zulaufenden Schlauchs wieder durch den Durchführkanal hindurch von dem Halter zu entfernen.

Auch das im Sinne der Erfindung vorgeschlagene Schlauchziehmittel kann nahezu beliebig ausgestaltet sein.

Zweckmäßigerweise weist das Schlauchziehmittel einen Rückziehantrieb auf, so dass der an dem Halter durch die von außen angreifende Schlauchklemme geklemmte zulaufende Schlauch entgegen die Einbringrichtung bzw. die Schlauchzustellrichtung und damit in Schlauchrückstellrichtung gezogen werden kann.

Zum Einbringen von immer ausreichend hohen Zugkräften ist es vorteilhaft, wenn das Schlauchziehmittel eine Klemme aufweist, welche gegenüber dem Schlauchzughaltemittel angetrieben ist, bevorzugt über eine Kopplung mit dem Übergabemittel.

Speziell, wenn die Klemme an dem Übergabemittel befestigt ist, kann die Klemme mittels des Übergabemittels translatorisch gegenüber dem Halter bewegt werden.

Darüber hinaus ist es vorteilhaft, wenn das Schlauchziehmittel dazu eingerichtet ist, in Zusammenwirken mit dem Schlauchzughaltemittel eine Dehnung von 1% bis 10% auf den Übergang von Schlauchstück zu Restschlauch zwischen dem Schlauchziehmittel und dem Schlauchzughaltemittel aufzuprägen, insbesondere von 2% bis 6%, vor allem von etwa 4%.

Speziell bei einer derart ausgewählten Dehnung in dem designierten Trennbereich des zulaufenden Schlauchs kann die Entstehung von Schlauchmaterialrestpartikeln beim Trennen des abzutrennenden Schlauchstücks von dem Restschlauch des zulaufenden Schlauchs besonders effektiv vermieden werden.

Insofern ist auch eine entsprechende Verfahrensführung vorteilhaft, bei welcher das Längsziehen des Schlauchs mit einer Längsdehnung zwischen 1% und 10% durchgeführt wird, bevorzugt von 2% bis 6%, besonders bevorzugt von etwa 4%.

Zum Abtrennen des Schlauchstücks von dem zulaufenden Schlauch können unterschiedliche Mittel eingesetzt werden, beispielsweise eine Drahtschlaufe oder dergleichen.

Eine im Sinne der Erfindung auch ohne die übrigen Merkmale der Erfindung besonders saubere Abtrennung des Schlauchstücks von dem zulaufenden Schlauch kann gewährleistet werden, wenn die Schlauchübergabestation ein Messer aufweist, welches angetrieben ist, und zwar von einer Bereitschaftsposition durch den zulaufenden Schlauch hindurch.

Durch ein derart angetriebenes Messer kann der Schlauch konstruktiv einfach durchgeschnitten werden.

Somit wird die Aufgabe der Erfindung auch von einer Anlage zum Herstellen eines Beutels für medizinische Zwecke umfassend eine Schlauchübergabestation mit einem Übergabemittel zum Übergeben eines der Schlauchübergabestation zulaufenden Schlauchs auf einen Halter und zum dortigen Abtrennen eines an den Halter übergebenen Schlauchstücks von einem Restschlauch des zulaufenden Schlauchs gelöst, um das Schlauchstück anschließend mit zwei Folienlagen zum Bilden eines Zugangs an dem Beutel verbindbar machen zu können, wobei die Schlauchübergabestation ein Messer aufweist, welches von einer Bereitschaftsposition durch den zulaufenden Schlauch hindurch angetrieben ist, wobei das Messer nach dem Trennen sich von dem Restschlauch freizustellen angetrieben ist, und zwar längs in Schlauchrichtung oder mit einer Bewegungskomponente längs in Schlauchrichtung.

Dadurch, dass das Messer nach dem Trennen gegenüber dem zulaufenden Schlauch, freigestellt werden kann, kann die Gefahr verringert werden, dass Schlauchmaterialrestpartikel erst durch eine zusätzliche Berührung des Messers mit dem Schlauch erzeugt werden.

Vorteilhafterweise wird das Messer hierbei zumindest von dem abgetrennten Schlauchstück freigestellt, bevorzugt jedoch sowohl von diesem Schlauchstück als auch von dem Restschlauch des zulaufenden Schlauchs.

Das Messer kann aus einem Zuführbereich des zulaufenden Schlauchs sehr schnell wieder entfernt werden, wenn das Messer einen Kippantrieb aufweist. Somit kann dem Halter den zulaufenden Schlauch wieder schnellstmöglich zugestellt werden.

Auch deshalb ist es vorteilhaft, wenn das Messer zum Freistellen gekippt wird.

Speziell ein Wiedereinbringen des zulaufenden Schlauchs in den Trennbereich für einen nächsten Abtrennvorgang kann schneller erfolgen, wenn der Kippantrieb das Messer von dem Übergabemittel wegkippend eingerichtet ist.

Ist das Messer sich schräg durch den Schlauch bewegend angetrieben, kann die Trennung des zulaufenden Schlauchs aufgrund einer vorteilhaften Schnittführung nochmals verbessert werden.

Der zulaufende Schlauch kann nochmals günstiger durchtrennt werden, wenn das Messer eine Dicke von maximal 1 mm aufweist, vor allem von maximal 0,8 mm, bevorzugt von maximal 0,5 mm, besonders bevorzugt von unter 0,2 mm.

Es wurde gefunden, dass die Gefahr einer Kontaminierung des zulaufenden Schlauchs durch Schlauchmaterialrestpartikel weiter reduziert werden kann, je dünner das Messer hinsichtlich seiner Dicke ausgestaltet ist.

Hierbei können bereits sehr gut verbesserte Schnittergebnisse erzielt werden, wenn das Messer eine Dicke von maximal 1 mm aufweist, zumal das Messer bei dieser Dicke sehr langlebig ausgelegt ist.

Insbesondere die Schlauchübergabestation kann konstruktiv noch einfacher ausgelegt werden, wenn übergabemittelseits das Schlauchziehmittel und das Messer angeordnet sind, wohingegen halterseits das Schlauchzughaltemittel angeordnet ist.

Hinsichtlich des Verfahrens ist es vorteilhaft, wenn der Halter und das Übergabemittel voneinander entfernt werden, insbesondere wenn das Übergabemittel zurückgezogen wird, und dabei der zulaufende Schlauch an der designierten Stelle zum Trennen in die Länge gezogen wird.

Verfahrenstechnisch ist es weiter vorteilhaft, wenn das Abtrennen mittels eines Messers erfolgt, welches während der auf die designierte Stelle zum Abtrennen wirkenden Zugkraft durch den zulaufenden Schlauch zugeführt wird, so dass der zulaufende Schlauch zumindest einerseits, bevorzugt beidseits, der abgetrennten Stelle der Zugkraft nachfolgt und sich nach dem Abtrennen eine Kluft ergibt, die breiter ist als eine Dicke des Messers.

Die Entstehung von unerwünschten Schlauchmaterialrestpartikeln kann signifikant reduziert werden, wenn das Messer zum Abtrennen auf eine Temperatur zwischen 40°C und 70 °C geheizt wird, vor allem zwischen 40 °C und 60 °C, insbesondere um 50 °C.

Durch die hier beschriebene Anlage bzw. das hier beschriebene Verfahren kann vorliegend insbesondere auch auf zusätzliche Hilfsmittel zum Durchtrennen des zulaufenden Schlauchs verzichtet werden, so dass zweckmäßigerweise das Abtrennen unter Meidung von alkoholischem Gleitmittel erfolgen kann.

Somit kann eine zusätzliche Kontaminierung des Zugangs des medizinischen Beutels reduziert werden.

Es versteht sich, dass die Merkmale der vorstehend bzw. in den Ansprüchen beschriebenen Lösungen gegebenenfalls auch kombiniert werden können, um die vorliegend erzielbaren Vorteile und Effekte entsprechend kumuliert umsetzen zu können.

Zusätzlich sind auch weitere Merkmale, Effekte und Vorteile vorliegender Erfindung anhand anliegender Zeichnung und an nachfolgender Beschreibung erläutert, in welchen beispielhaft eine Schlauchübergabestation an einer Anlage zum Herstellen eines medizinischen Beutels dargestellt und beschrieben ist.

In der Zeichnung zeigen:
- Figur 1: schematisch eine Anlage zum Herstellen eines Beutels für medizinische Zwecke in einem Bereich, in welchem eine Schlauchübergabestation angeordnet ist, welche halterseits ein Schlauchzughaltemittel und andererseits ein Schlauchziehmittel aufweist, um Zugkräfte in einen zulaufenden Schlauch in Schlauchlängsrichtung einbringen zu können;
- Figur 2: schematisch ein Verfahrensschritt an der der in der Figur 1 gezeigten Schlauchaufsteckstation, bei welchem ein auf eine Mandrene aufgesteckter Schlauch von radial außen geklemmt und anschließend axial vorgespannt wird;
- Figur 3: schematisch ein weiterer Verfahrensschritt an der in der Figur 1 gezeigten Schlauchaufsteckstation, bei welchem der von radial außen geklemmte und axial vorgespannte Schlauch geschnitten wird;
- Figur 4: schematisch ein nächster Verfahrensschritt an der in der Figur 1 gezeigten Schlauchübergabestation, bei welchem die Schlauchziehmittel und das Messer axial zurückgefahren werden;
- Figur 5: schematisch ein nachfolgender Verfahrensschritt an der in der Figur 1 gezeigten Schlauchübergabestation, bei welchem das Messer nach radial unten weggeklappt wird;
- Figur 6: schematisch ein darauffolgender Verfahrensschritt an der in der Figur 1 gezeigten Schlauchübergabestation, bei welchem das Messer translatorisch nach radial unten verfahren wird;
- Figur 7: schematisch ein weiterer Verfahrensschritt an der in der Figur 1 gezeigten Schlauchübergabestation, bei welchem das Messer in Vorbereitung eines nächsten Schnitts wieder entlang der Kreisbahn nach oben in eine Bereitschaftsposition geklappt wird; und
- Figur 8: schematisch eine Ansicht der in der Figur 1 gezeigten Schlauchübergabestation mit aktivierten Luftdüsen.

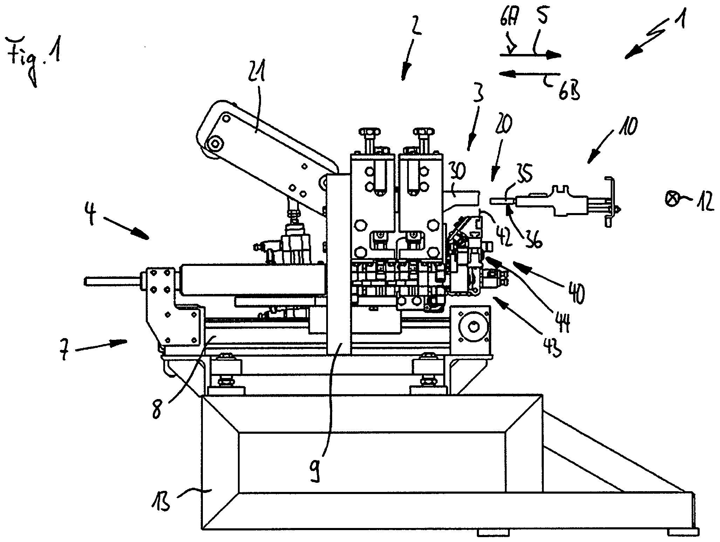

Die in der Figur 1 zumindest teilweise gezeigte Anlage 1 zum Herstellen eines Beutels (nicht gezeigt) für medizinische Zwecke umfasst unter anderem eine Schlauchübergabestation 2, welche gemäß der Darstellung nach der Figur 1 schematisch in einer Seitenansicht gezeigt ist.

Diese Schlauchübergabestation 2 verfügt über ein Übergabemittel 3, welches mittels einer Verfahrmechanik 4 translatorisch in Einbringrichtung 5 bzw. Schlauchzustellrichtung 6A oder entgegen der Einbringrichtung 5 in Schlauchrückstellrichtung 6B verlagert werden kann.

Diese Verfahrmechanik 4 weist zumindest eine Linearverfahreinrichtung 7 mit einer Lineartraverse 8 und einen Schlittenteil 9 auf, an welchem letztendlich die Übergabemittel 3 aufgehangen sind.

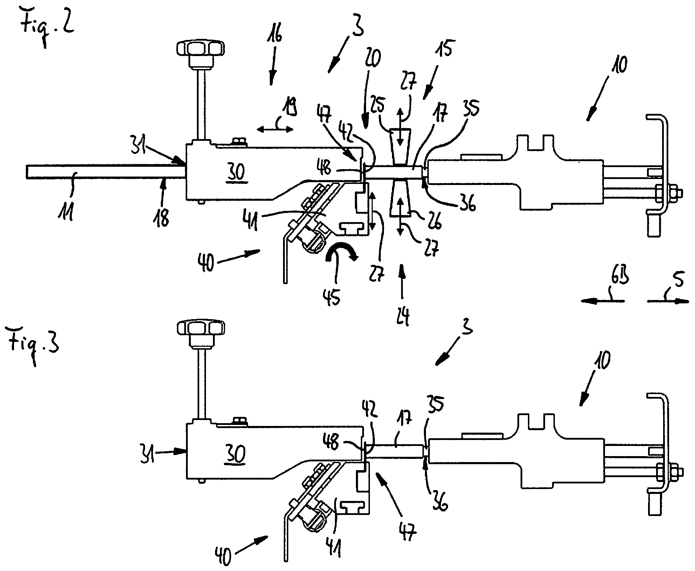

In Einbringrichtung 5 bzw. Schlauchzustellrichtung 6A gesehen vor diesen Übergabemitteln 3 befindet sich ein Halter 10, an welchem ein zulaufender Schlauch 11 (siehe Figur 2) übergeben werden soll.

Der Halter 10 läuft hierbei bedarfsweise in Bewegungsumlaufrichtung 12 vor der Schlauchübergabestation 2 bzw. vor den Übergabemitteln 3 ein und wird hierbei derart gestoppt, dass der zulaufende Schlauch 11 von der Schlauchübergabestation 2 im Sinne der Erfindung an den Halter 10 übergeben werden kann.

Der Halter 10 ist hierzu an einem umlaufenden Förderband (nicht gezeigt) einer Fördereinrichtung (nicht gezeigt) der Anlage 1 geführt, wobei auf die diesbezügliche Fördereinrichtung nicht weiter eingegangen wird, da diese aus dem Stand der Technik hinlänglich bekannt ist.

Die gesamte Anlage 1 ist mit einem Gestell 13 fest mit einem Boden (nicht gesondert beziffert) verbunden.

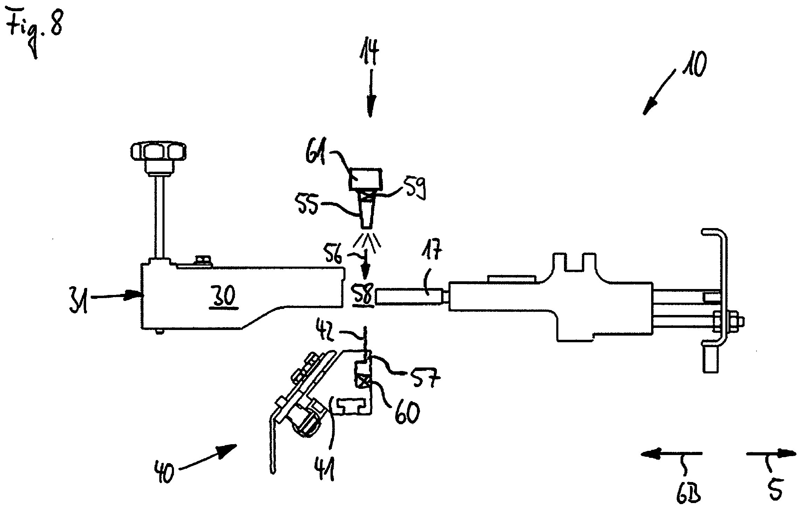

Wie nachfolgend noch detaillierter im Zusammenhang mit der Figur 8 gezeigt ist, zeichnet sich die Schlauchübergabestation 2 noch durch eine Luftzufuhr- bzw. Luftabzugseinrichtung 14 aus, mittels welcher eine erzwungene Luftströmung bzw. Luftzirkulation an der Schlauchübergabestation 2 erzielt werden kann.

Wie speziell gemäß der Darstellung nach der Figur 2 gut ersichtlich ist, zeichnet sich die vorliegende Schlauchübergabestation 2 jedenfalls halterseits durch ein Schlauchzughaltemittel 15 und andererseits durch ein Schlauchziehmittel 16 aus, welche dazu eingerichtet sind, zwischen einem abgetrennten Schlauchstück 17 und einem Restschlauch 18 des zulaufenden Schlauchs 11 Zugkräfte 19 einzubringen.

Insbesondere werden diese Zugkräfte 19 im Bereich einer Schnittstelle und während des Abtrennens des Restschlauchs 18 von dem abzutrennenden Schlauchstück 17 eingebracht, um hierdurch an einer Trenn- bzw. Schnittstelle 20 einen verbesserten Schnitt bzw. eine verbesserte Schnittführung zu erzielen, wodurch weniger bzw. signifikant weniger Schlauchmaterialrestpartikel (hier nicht gezeigt) erzeugt werden, welche insbesondere den späteren Zugang und damit auch den Inhalt des medizinischen Beutels kontaminieren können.

Zum Aufbringen der Zugkräfte 19 weist das Schlauchziehmittel 16 einen Rückziehantrieb 21 auf, welcher dem Übergabemittel 3 zugeordnet ist, sodass dieser Rückziehantrieb 21 gemeinsam mit dem Übergabemittel 3 mittels der Linearverfahreinrichtung 7 bewegt werden kann.

Insbesondere mittels des Schlauchziehmittels 16 in Zusammenwirken mit dem Schlauchzughaltemittel 15 kann eine Dehnung des zulaufenden Schlauchs 11 speziell im Bereich der Schnittstelle 20 von 1 % bis 10 % erzielt werden.

Besonders vorteilhaft ist eine Dehnung von etwa 4 %, da sich herausgestellt hat, dass hierbei der Schnitt derart erfolgen kann, dass kaum mehr Schlauchmaterialrestpartikel beim Schneiden erzeugt werden.

Die dem Halter 10 zugeordneten Schlauchzughaltemittel 15 umfassen zumindest in diesem Ausführungsbeispiel eine Klemmeinrichtung (nicht gesondert beziffert) in Gestalt einer von radial außen angreifenden Schlauchklemme 24, welche insbesondere zwei Klemmelemente 25 und 26 aufweist, die dem abzutrennenden Schlauchstück 17 von radial außen zugestellt werden können, wobei die beiden Klemmelemente 25 und 26 bezogen auf die Einbringrichtung 5 quer, das heißt in radialer Richtung 27, beweglich gelagert sind.

Das Schlauchziehmittel 16 hingegen ist zumindest in diesem Ausführungsbeispiel durch hier nicht weiter gezeigte Fixierelemente und/oder durch den Rückziehantrieb 21 ergänzt, welche dem Übergabemittel 3 zugeordneten sind.

Insofern kann einerseits das an dem Halter 10 gehalterte abzutrennende Schlauchstück 17 an dem Halter 10 und andererseits der Restschlauch 18 des zulaufenden Schlauchs 11 an dem Übergabemittel 3 fixiert bzw. gehaltert werden, wobei durch derart ausgestaltete Übergabemittel 3 die vorliegenden Schlauchziehmittel 16 konstruktiv besonders einfach realisiert sind.

Der zulaufende Schlauch 11 ist vorliegend besonders präzise an dem Übergabemittel 3 seitlich geführt, da das Übergabemittel 3 einen Grundkörper 30 aufweist, welcher zumindest einen Durchführkanal 31 für den zulaufenden Schlauch 11 aufweist, durch welchen hindurch der zulaufende Schlauch 11 in Zustellrichtung 5 geführt und somit auch relativ gegenüber dem Grundkörper 30 bewegt werden kann.

Damit dann halterseits der zulaufende Schlauch 11 bzw. das abzutrennende Schlauchstück 17 betriebssicher fixiert werden kann, besitzt der Halter 10 speziell in diesem Ausführungsbeispiel einen Dorn 35, welcher hier sogleich als dornförmige Zentralelektrode 36 ausgestaltet ist.

Vorteilhafterweise weist der Dorn 35 bzw. die dornförmige Zentralelektrode 36 einen Außendurchmesser (nicht gesondert beziffert) auf, welcher mit dem Innendurchmesser (ebenfalls nicht explizit beziffert) des zulaufenden Schlauchs 11 derart komplementär ist, dass der in Schlauchzustellrichtung 6A geförderte zulaufende Schlauch 11 betriebssicher auf den Dorn 35 bzw. auf die dornförmige Zentralelektrode 36 aufgesteckt werden kann. Insbesondere aufgrund dieser speziellen Ausführungsform kann vorliegend hinsichtlich der Schlauchübergabestation 2 auch von einer Schlauchaufsteckstation (nicht zusätzlich beziffert) gesprochen werden.

Genauso verhält es sich hinsichtlich des Übergabemittels 3, wobei vorliegend dieses ein Aufsteckmittel (nicht zusätzlich beziffert) zum Aufstecken des zulaufenden Schlauchs. 11 auf den Halter 10 ist.

Der Schlauchübergabestation 2 ist ferner eine Trenn- bzw. Schneideinrichtung 40 zugeordnet, welche gemeinsam mit dem Übergabemittel 3 an der Linearverfahreinrichtung 7 angeordnet ist.

Die Trenn- bzw. Schneideinrichtung 40 weist in diesem Ausführungsbeispiel einen Schneidkopf 41 auf, der ein Messer 42 zum Schneiden des zulaufenden Schlauchs 11 trägt.

Die Trenn- bzw. Schneideinrichtung 40 zeichnet sich des Weiteren dadurch aus, dass sie einerseits einen Hebe-Senkantrieb 43 und andererseits zusätzlich einen Kippantrieb 44 aufweist.

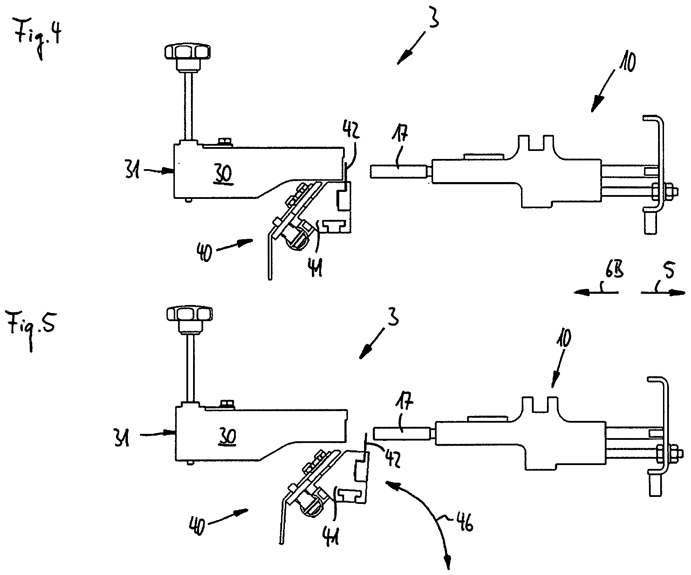

Somit kann das Messer 42 gegenüber der Einbringrichtung 5 des zulaufenden Schlauchs 11 nicht nur in radialer Richtung 27 translatorisch bewegt werden, sondern das Messer kann mittels des Kippantriebs 44 gemäß Pfeil 45 entlang einer Kreisbahn 46 (siehe insbesondere Figur 5) geführt werden, um hierdurch nach unten von der Schnittstelle 20 entfernt zu werden.

Das Messer 42 hat in diesem Ausführungsbeispiel beispielhaft eine Dicke von lediglich 0,2 mm, was sich wiederum sehr günstig auf die Vermeidung von Schlauchmaterialrestpartikeln beim Trennen des zulaufenden Schlauchs 11 auswirkt.

Gemäß der Darstellung nach der Figur 2 ist die Schlauchübergabestation 2 in einem Verfahrenszustand gezeigt, bei welchem der zulaufende Schlauch 11 dem Halter 10 bereits zugeführt ist, wobei das abzutrennende Schlauchstück 17 bzw. das bereits abgetrennte Schlauchstück 17 auf den Dorn 35 des Halters 10 aufgesteckt ist.

Auch ist der zulaufende Schlauch 11 bzw. das bereits abgetrennte Schlauchstück 17 durch das Schlauchzughaltemittel 15 in Gestalt der von radial außen angreifenden Schlauchklemme 24 gehalten, wobei der zulaufende Schlauch 11 mittels der Schlauchziehmittel 16 längs gezogen und das abzutrennende Schlauchstück 17 bereits von dem Restschlauch 18 des zulaufenden Schlauchs 11 abgetrennt ist.

Das Messer 42 wurde, während auf die designierte Schnittstelle 20 die Zugkräfte 19 bereits wirken, durch den Schlauch 11 geführt, sodass der zulaufende Schlauch 11 zumindest einerseits, bevorzugt aber beiderseits, an der abgetrennten Stelle 20 den Zugkräften 19 nachfolgt und sich nach dem Abtrennen eine Kluft 48 ergibt, welche in jedem Fall breiter ist als die Dicke des Messers 42.

Während gemäß der Darstellung nach Figur 2 die Schlauchübergabestation 2 noch mit aktivierter Schlauchklemme 24 und einem gerade durchtrennten zulaufenden Schlauch 11 dargestellt ist, ist gemäß der Darstellung nach Figur 3 bereits ein nächster Verfahrensschritt der Schlauchübergabestation 2 gezeigt, bei welchem die Klemmelemente 25 und 26 der von radial außen angreifenden Schlauchklemme 24 wieder nach radial außen und zurückverlagert sind. Das Messer 42 befindet sich hierbei jedoch noch in Schneidposition 47.

Gemäß der Darstellung nach der Figur 4 ist das Messer 42 nach dem Durchtrennen des zulaufenden Schlauchs 11 sowohl von dem abgetrennten Schlauchstück 17 als auch von dem Restschlauch 18 freigestellt, sowohl einerseits mittels einer translatorischen Bewegung in Schlauchrückstellrichtung 6B der Übergabemittel 3 und andererseits mittels einer Kippbewegung des Messers entlang der Kreisbahn 46 gemäß der Darstellung nach der Figur 5.

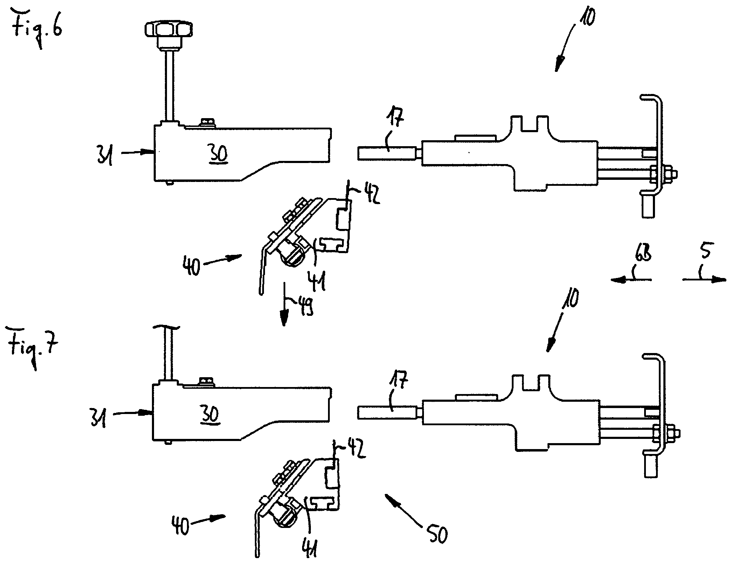

Gemäß der Darstellung nach der Figur 6 ist die Schlauchübergabestation 2 in einem nächsten Verfahrensschritt gezeigt, bei welchem das Messer 42 bzw. der gesamte Schneidkopf 41 mittels des Hebe- bzw. Senkantriebs 43 zusätzlich in Abwärtsrichtung 49 nach unten abgesenkt ist, sodass das Messer 42 vollständig aus dem Bereich der Schnittstelle 20 herausbewegt ist.

Letztlich wird gemäß der Darstellung nach der Figur 7 das Messer 42 wieder in eine Bereitschaftsposition 50 hineingekippt, sodass der zulaufende Schlauch 11 einem weiteren Halter 10 gemäß der Schlauchzustellrichtung 6A zugeführt werden kann.

Es versteht sich, dass nach dem Freistellen des Messers 42 von dem abgetrennten Schlauchstück 17 der Halter 10 einer weiteren Bearbeitungsstation (hier nicht gezeigt) der Anlage 1 zugestellt werden kann, und dass das noch an dem Halter 10 gehaltene abgetrennte Schlauchstück 17 zur Weiterverarbeitung an dieser weiteren Bearbeitungsstation bereitgestellt ist.

Gemäß der Darstellung nach der Figur 8 ist die Luftzufuhr- bzw. Luftabzugseinrichtung 14 in einem aktivierten Zustand gezeigt.

Die Luftzufuhr- bzw. Luftabzugseinrichtung 14 umfasst zumindest in diesem Ausführungsbeispiel eine erste Luftdüse 55 zum Ausblasen von Luft in einen Bereich der designierten Stelle 20 hinein, so dass eine erzwungene Luftströmung 56 diese designierte Stelle 20 um- bzw. durchströmt.

Diese erste Luftdüse 55 ist oberhalb des Grundkörpers 30 des Übergabemittels 3 positioniert.

Ferner ist diese erste Luftdüse 55 zwischen dem Übergabemittel 3 und dem Halter 10 angeordnet.

Die Luftzufuhr- bzw. Luftabzugseinrichtung 14 umfasst in diesem Ausführungsbeispiel auch eine weitere Luftdüse 57 zum Einsaugen bzw. Absaugen von Luft aus dem Bereich der designierten Stelle 20 heraus, so dass die erzwungene Luftströmung 56 von der weiteren Luftdüse 57 abgesaugt und gegebenenfalls noch verstärkt wird.

Es ist auch denkbar, dass die erzwungene Luftströmung 56 ausschließlich von der weiteren Luftdüse 57 erzeugt wird, beispielsweise, wenn die erste Luftdüse 55 außer Funktion ist oder erst gar nicht vorgesehen sein sollte.

Die weitere Luftdüse 57 ist platzsparend direkt in dem gemäß der Kreisbahn 46 (siehe Figur 5) klappbaren Schneidkopf 41 der Trenn- bzw. Schneideinrichtung 40 integriert.

Insofern ist die weitere Luftdüse 57 konstruktiv einfach unterhalb des Grundkörpers 30 des Übergabemittels 3 positioniert.

Ebenso ist die weitere Luftdüse 57 sogleich zwischen dem Übergabemittel 3 und dem Halter 10 angeordnet.

Insgesamt betrachtet liegt der jeweilige Wirkbereich 58 (nur exemplarisch beziffert) der zwei Luftdüsen 55 und 57 insbesondere auf Höhe der beim Trennen des zulaufenden Schlauchs 11 entstehenden Kluft 48 bzw. der designierten Stelle 20.

Der wesentliche Vorteil dieser Luftzufuhr- bzw. Luftabzugseinrichtung 14 ist darin zu sehen, dass mittels der ersten Luftdüse 55 Schlauchmaterialrestpartikel von dem abgetrennten Schlauchstück 17 fortgeblasen werden, sofern derartige Schlauchmaterialpartikel beim Abtrennen des Schlauchstücks 17 erzeugt wurden.

Jedoch werden diese Schlauchmaterialpartikel nicht nur einfach aus dem Bereich der designierten Stelle 20 fortgeblasen, sondern vorteilhafterweise mittels der weiteren Luftdüse 57 sogleich auch noch ein- bzw. abgesaugt, so dass die Schlauchmaterialpartikel idealerweise nicht oder lediglich in extrem reduzierter Anzahl in die Umgebung der Schlauchübergabestation 2 gelangen können.

Vorteilhafterweise umfassen die Luftdüsen 55 und 57 sogleich jeweils ein Druckdifferenzmittel 59 bzw. 60 zum Erzeugen der Luftströmung 56, wobei das erste Druckdifferenzmittel 59 eine Gebläseeinrichtung (nicht gesondert beziffert) und das zweite Druckdifferenzmittel 60 eine Absaugeinrichtung (nicht gesondert beziffert) umfassen.

Um insbesondere das abgetrennte Schlauchstück 17 an der Schlauchübergabestation 2 auch noch sterilisieren zu können, weist die Schlauchübergabestation 2 des Weiteren noch eine Luftionisiereinrichtung 61 auf, welche in diesem Ausführungsbeispiel der ersten Luftdüse 55 zugeordnet ist, so dass die hieraus ausströmende Luft bereits ionisiert ist.

An dieser Stelle sei explizit darauf hingewiesen, dass die Merkmale der vorstehend bzw. in den Ansprüchen und/oder Figuren beschriebenen Lösungen gegebenenfalls auch kombiniert werden können, um die erläuterten Merkmale, Effekte und Vorteile entsprechend kumuliert umsetzen bzw. erzielen zu können.

Es versteht sich, dass es sich bei dem vorstehend erläuterten Ausführungsbeispiel lediglich um eine erste Ausgestaltung der erfindungsgemäßen Anlage bzw. Schlauchübergabestation und des erfindungsgemäßen Verfahrens handelt, insofern beschränkt sich die Ausgestaltung der Erfindung nicht auf dieses Ausführungsbeispiel.

Sämtliche in den Anmeldeunterlagen offenbarten Merkmale werden als erfindungswesentlich beansprucht, sofern sie einzeln oder in Kombination gegenüber dem Stand der Technik neu sind.

### Bezugszeichenliste

- 1: Anlage
- 2: Schlauchübergabestation
- 3: Übergabemittel
- 4: Verfahrmechanik
- 5: Einbringrichtung
- 6A: Schlauchzustellrichtung
- 6B: Schlauchrückstellrichtung
- 7: Linearverfahreinrichtung
- 8: Lineartraverse
- 9: Schlittenteil
- 10: Halter
- 11: zulaufender Schlauch
- 12: Bewegungsumlaufrichtung
- 13: Gestell
- 14: Luftzufuhr- bzw. Luftabzugseinrichtung
- 15: Schlauchzughaltemittel
- 16: Schlauchziehmittel
- 17: übergebenes, abgetrenntes bzw. abzutrennendes Schlauchstück
- 18: Restschlauch
- 19: Zugkräfte
- 20: Schnittstelle bzw. designierte Stelle
- 21: Rückziehantrieb
- 24: von radial außen angreifende Schlauchklemme
- 25: erstes Klemmelement
- 26: zweites Klemmelement
- 27: radiale Richtung
- 30: Grundkörper
- 31: Durchführkanal
- 35: Dorn
- 36: dornförmige Zentralelektrode
- 40: Trenn- bzw. Schneideinrichtung
- 41: Schneidkopf bzw. Halteeinrichtung
- 42: Messer bzw. Trennmittel
- 43: Hebe-Senkantrieb
- 44: Kippantrieb
- 45: Pfeil
- 46: Kreisbahn
- 47: Schneidposition
- 48: Kluft
- 49: Abwärtsrichtung
- 50: Bereitschaftsposition
- 55: erste Luftdüse
- 56: Luftströmung
- 57: weitere Luftdüse
- 58: Wirkbereich
- 59: erstes Druckdifferenzmittel
- 60: weiteres Druckdifferenzmittel
- 61: Luftionisiereinrichtung

## Patentansprüche

1. Anlage (1) zum Herstellen eines Beutels für medizinische Zwecke umfassend eine Schlauchübergabestation (2) mit einem Übergabemittel (3) zum Übergeben eines der Schlauchübergabestation (2) zulaufenden Schlauchs (11) an einen Halter (10)
und zum dortigen Abtrennen eines an den Halter (10) übergebenen Schlauchstücks (17) von einem Restschlauch (18) des zulaufenden Schlauchs (11), um das abgetrennte Schlauchstück (17) anschließend mit zwei Folienlagen zum Bilden eines Zugangs an dem Beutel verbindbar machen zu können,
***dadurch gekennzeichnet, dass***
die Schlauchübergabestation (2) wenigstens eine Luftdüse (55, 57) umfasst, wobei ein Wirkbereich (58) der wenigstens einen Luftdüse (55, 57) auf eine designierte Stelle (20) zum Abtrennen gerichtet ist, so dass eine durch ausgeblasene Luft und/oder eingesaugte Luft erzwungene Luftströmung (56) an der designierten Stelle (20) zum Abtrennen einstellbar ist, um beim Abtrennen des Schlauchstücks (17) von dem zulaufenden Schlauch (11) erzeugte Schlauchmaterialrestpartikel von der designierten Stelle (20) bzw. von dem Schlauchstück (17) und auch noch von dem Restschlauch (18) durch die erzwungene Luftströmung (56) zu entfernen.

2. Anlage (1) nach Anspruch 1,
***dadurch gekennzeichnet, dass***
die wenigstens eine Luftdüse (55, 57) ein Druckdifferenzmittel (59, 60) zum Erzeugen eines Überdrucks und/oder eines Unterdrucks an der Luftdüse (55, 57) aufweist, so dass im Betrieb des Druckdifferenzmittels (59, 60) eine durch ausgeblasene Luft und/oder eingesaugte Luft erzwungene Luftströmung (56) an der designierten Stelle (20) zum Abtrennen einstellbar ist.

3. Anlage (1) nach Anspruch 1 oder 2,
***dadurch gekennzeichnet, dass***
zwei Luftdüsen (55, 57) vorgesehen sind, wovon eine erste Luftdüse (55) zum Ausblasen von Luft und eine zweite Luftdüse (57) zum Einsaugen von Luft eingerichtet ist.

4. Anlage (1) nach einem der vorstehenden Ansprüche, ***dadurch***
***gekennzeichnet, dass*** die designierte Stelle (20) zum Abtrennen zwischen den Luftdüsen (55, 57) angeordnet ist.

5. Anlage (1) nach einem der vorstehenden Ansprüche,
***dadurch gekennzeichnet, dass***
die zweite Luftdüse (57) zum Einsaugen von Luft schwenkbar an der Schlauchübergabestation (2) angeordnet ist.

6. Anlage (1) nach einem der vorstehenden Ansprüche,
***dadurch gekennzeichnet,***
***dass*** die zweite Luftdüse (57) zum Einsaugen von Luft an einer schwenkbaren Halteeinrichtung (41) zum Halten eines Trennmittels (42) zum Trennen des zulaufenden Schlauchs (11) angeordnet ist.

7. Anlage (1) nach einem der vorstehenden Ansprüche,
**dadurch gekennzeichnet, dass**
die Schlauchübergabestation (2) halterseits ein Schlauchzughaltemittel (15) und andererseits ein Schlauchziehmittel (16) aufweist, welche dazu eingerichtet sind, zwischen dem abgetrennten Schlauchstück (17) und dem Restschlauch (18) des zulaufenden Schlauchs (11) Zugkräfte (19) einzubringen, während das Abtrennen erfolgt.

8. Anlage (1) nach einem der vorstehenden Ansprüche,
**dadurch gekennzeichnet, dass**
der Halter (10) einen Dorn (35) aufweist, insbesondere eine dornförmige Zentralelektrode (36), und/oder
eine von außen angreifende Schlauchklemme (24) aufweist, und/oder in Einbringrichtung (5) des zulaufenden Schlauchs (11) fixiert ist.

9. Anlage (1) nach einem der vorstehenden Ansprüche,
**dadurch gekennzeichnet, dass**
das Übergabemittel (3) einen Durchführkanal (31) für den zulaufenden Schlauch (11) aufweist, und/oder
in Einbringrichtung (5) vor-und rückfahrbar ist, und/oder dazu eingerichtet ist, den Durchführkanal (31) nah an den Halter (10) zu verfahren, dann den zulaufenden Schlauch (11) zumindest mit dem abzutrennenden Schlauchstück (17) zu übergeben, und nach Abtrennen des Schlauchstücks (17) den Restschlauch (18) des zulaufenden Schlauchs (11) wieder durch den Durchführkanal hindurch von dem Halter (10) zu entfernen.

10. Anlage (1) nach einem der Ansprüche 7 bis 9,
**dadurch gekennzeichnet, dass**
das Schlauchziehmittel (16) einen Rückziehantrieb (21) und/oder eine Klemme aufweist, welche gegenüber dem Schlauchzughaltemittel (15) angetrieben ist, bevorzugt über eine Kopplung mit dem Übergabemittel (3), und/oder
dazu eingerichtet ist, in Zusammenwirken mit dem Schlauchzughaltemittel (15) eine Dehnung von 1 % bis 10 % auf den Übergang von abzutrennendem Schlauchstück (17) zu Restschlauch (18) zwischen dem Schlauchziehmittel (16) und dem Schlauchzughaltemittel (15) aufzuprägen, insbesondere von 2 % bis 6 %, vor allem von etwa 4 %.

11. Anlage (1) nach einem der vorstehenden Ansprüche,
**dadurch gekennzeichnet, dass**
die Schlauchübergabestation (2) ein Messer (42) aufweist, welches angetrieben ist, und zwar von einer Bereitschaftsposition (50) durch den zulaufenden Schlauch (11) hindurch.

12. Anlage (1) nach Anspruch 11,
**dadurch gekennzeichnet, dass**
das Messer (42) von der Bereitschaftsposition (50) durch den zulaufenden Schlauch (11) hindurch angetrieben ist, wobei das Messer (42) nach dem Trennen sich von dem Restschlauch (18) freizustellen angetrieben ist, und zwar längs in Schlauchrichtung oder mit einer Bewegungskomponente (53) längs in Schlauchrichtung.

13. Anlage (1) nach Anspruch 11 oder 12,
**dadurch gekennzeichnet, dass**
das Messer (42) einen Kippantrieb (44) aufweist.

14. Anlage (1) nach Anspruch 13,
**dadurch gekennzeichnet, dass**
der Kippantrieb (44) das Messer (42) von dem Übergabemittel (3) wegkippend eingerichtet ist.

15. Anlage (1) nach einem der Ansprüche 11 bis 14,
**dadurch gekennzeichnet, dass**
das Messer (42) sich schräg durch den Schlauch (11) bewegend angetrieben ist, und/oder
das Messer (42) eine Dicke von maximal 1 mm aufweist, vor allem von maximal 0,8 mm, bevorzugt von maximal 0,5 mm, besonders bevorzugt von unter 0,2 mm.

16. Anlage (1) nach einem der Ansprüche 11 bis 15,
**dadurch gekennzeichnet, dass**
übergabemittelseits das Schlauchziehmittel (16) und das Messer (42) angeordnet sind, wohingegen halterseits das Schlauchzughaltemittel (15) angeordnet ist.

17. Verfahren zum Herstellen eines Beutels für medizinische Zwecke, bei welchem ein einer Schlauchübergabestation (2) zulaufender Schlauch mittels eines Übergabemittels (3) an einen Halter (10) zum dortigen Abtrennen eines an den Halter (10) übergebenen Schlauchstücks (11) von einem Restschlauch (18) des zulaufenden Schlauchs (11) übergeben wird, so dass das Schlauchstück (17) anschließend mit zwei Folienlagen zum Bilden eines Zugangs an dem Beutel verbunden wird, *insbesondere* unter Verwendung einer Anlage (1) nach einem der vorstehenden Ansprüche,
***dadurch gekennzeichnet, dass***
während des Abtrennens des Schlauchstücks (17) von dem Restschlauch (18) des zulaufenden Schlauchs (11) wenigstens eine Luftdüse (55, 57) mit einem Druckdifferenzmittel (59, 60) zum Erzeugen eines Überdrucks und/oder eines Unterdrucks an der Luftdüse (55, 57) betrieben wird, wobei ein Wirkbereich (58) der wenigstens einen Luftdüse (55, 57) auf die designierte Stelle (20) zum Abtrennen gerichtet wird, und wobei das Druckdifferenzmittel (59, 60) während des Abtrennens derart betrieben wird, dass eine durch ausgeblasene Luft und/oder eingesaugte Luft erzwungene Luftströmung an der designierten Stelle (20) zum Abtrennen eingestellt wird, um beim Abtrennen des Schlauchstücks (17) von dem zulaufenden Schlauch (11) erzeugte Schlauchmaterialrestpartikel von der designierten Stelle (20) bzw. von dem Schlauchstück (17) und auch noch von dem Restschlauch (18) durch die erzwungene Luftströmung (56) zu entfernen.

18. Verfahren nach Anspruch 17,
***dadurch gekennzeichnet, dass***
Luft durch eine erste Luftdüse (55) ausgeblasen und Luft durch eine weitere Luftdüse (57) eingesaugt wird.

19. Verfahren nach Anspruch 17 oder 18,
***dadurch gekennzeichnet, dass***
eine Luftionisiereinrichtung zum Ionisieren von zu der wenigstens einen Luftdüse zuzuführender Luft betrieben wird und ionisierte Luft durch die wenigstens eine Luftdüse (55, 57) ausgeblasen wird.

20. Verfahren nach Anspruch 17 bis 19,
**dadurch gekennzeichnet, dass**
die folgenden Schritte durchgeführt werden:
Zuführen des zulaufenden Schlauchs (11) zum Halter (10);
zugfestes Halten des zulaufenden Schlauchs (11) an dem abzutrennenden Schlauchstück (17) auf Seiten des Halters (10) mittels eines Schlauchzughaltemittels (15);
c. Längsziehen des zulaufenden Schlauchs (11) zwischen dem Schlauchzughaltemittel (15) und einer Zuführung, sodass Zugkräfte auf die designierte Stelle (20) zum Abtrennen wirken; und
d. Abtrennen des Schlauchstücks (17) von dem Restschlauch (18) des zulaufenden Schlauchs (11).

21. Verfahren nach einem der Ansprüche 17 bis 20,
**dadurch gekennzeichnet, dass**
das Zuführen des Schlauchs (11) durch einen Durchführkanal (31) erfolgt, und/oder
das Zuführen des zulaufenden Schlauchs (11) auf einen Dorn (35) erfolgt, insbesondere auf eine Zentralelektrode (36).

22. Verfahren nach einem der Ansprüche 17 bis 21,
**dadurch gekennzeichnet, dass**
der zulaufende Schlauch (11) an einem abzutrennenden Schlauchstück (17) mit dem Halter (10) gegen Zurückziehen fixiert wird, vor allem von außen festgeklemmt wird.

23. Verfahren nach einem der Ansprüche 17 bis 22,
**dadurch gekennzeichnet, dass**
der Halter (10) und das Übergabemittel (3) voneinander entfernt werden, insbesondere das Übergabemittel (3) zurückgezogen wird, und dabei der zulaufende Schlauch (11) an der designierten Stelle (20) zum Abtrennen in die Länge gezogen wird.

24. Verfahren nach Anspruch 23,
**dadurch gekennzeichnet, dass**
das Abtrennen mittels eines Messers (42) erfolgt, welches während der auf die designierte Stelle (20) zum Abtrennen wirkenden Zugkraft durch den zulaufenden Schlauch (11) geführt wird, sodass der zulaufende Schlauch (11) zumindest einerseits, bevorzugt beiderseits, der abgetrennten Stelle der Zugkraft nachfolgt und sich nach dem Abtrennen eine Kluft (48) ergibt, die breiter ist als eine Dicke des Messers (42).

25. Verfahren nach Anspruch 24,
**dadurch gekennzeichnet, dass**
das Messer (42) nach dem Abtrennen von dem Schlauch (11) freigestellt wird, und zwar mittels einer Bewegung, die eine Komponente längs einer Zugkraftrichtung aufweist, und/oder
das Messer (42) zum Freistellen gekippt wird, und/oder das Messer (42) zum Abtrennen auf eine Temperatur zwischen 40 °C und 70 °C geheizt wird, vor allem zwischen 40 °C und 60 °C, insbesondere um 50 °C.

26. (nicht recherchiert)Verfahren nach einem der Ansprüche 17 bis 25,
**dadurch gekennzeichnet, dass**
das Längsziehen des Schlauchs (11) mit einer Längsdehnung zwischen 1 % und 10 % durchgeführt wird, bevorzugt von 2 % bis 6 %, besonders bevorzugt etwa 4 %.

27. Verfahren nach einem der Ansprüche 17 bis 26,
**dadurch gekennzeichnet, dass**
das Abtrennen unter Meidung von alkoholischem Gleitmittel erfolgt.

## Claims

1. Plant (1) for manufacturing a pouch for medical purposes, comprising a tubing transfer station (2) with a transfer means (3) for transferring a tubing (11) advancing to the tubing transfer station (2) to a holder (10)
and for separating there a piece (17) of tubing transferred to the holder (10) from a residual tubing (18) of the advancing tubing (11) in order to be able to subsequently make the separated piece (17) of tubing connectable to two sheets of film so as to form an access to the pouch;
**characterized in that**
the tubing transfer station (2) comprises at least one air nozzle (55, 57), an effective range (58) of the at least one air nozzle (55, 57) being directed at a designated site (20) for separating so that an air flow (56) forced by air which is blown out and/or air which is aspirated is adjustable for separating at the designated site (20) in order to remove tubing material residual particles from the designated site (20) or from the piece (17) of tubing, respectively, and also from the residual tubing (18) by means of the forced air flow (56) during separation of the piece (17) of tubing from the advancing tubing (11).

2. Plant (1) according to Claim 1,
**characterized in that**
the at least one air nozzle (55, 57) has a pressure difference means (59, 60) for creating an overpressure and/or a negative pressure at the air nozzle (55, 57) so that during operation of the pressure difference means (59, 60), an air flow (56) forced by air which is blown out and/or by air which is aspirated is adjustable for separating at the designated site (20).

3. Plant (1) according to Claim 1 or 2,
**characterized in that**
two air nozzles (55, 57) are provided, one of which (55) is adapted to blow out air and one of which (57) is adapted to aspirate air.

4. Plant (1) according to one of the above Claims, **characterized in that** the designated site (20) for separating is arranged between the air nozzles (55, 57).

5. Plant (1) according to one of the above Claims,
**characterized in that**
the second air nozzle (57) for aspirating air is arranged at the tubing transfer station (2) so as to be able to be slued.

6. Plant (1) according to one of the above Claims,
**characterized in that**
the second air nozzle (57) for aspirating air is arranged at a holding device (41), which can be slued, for holding a separating means (42) for separating the advancing tubing (11).

7. Plant (1) according to one of the above Claims,
**characterized in that**
the tubing transfer station (2) has, on its holding side, a tubing traction holding means (15) and, on the other side, a tubing pulling means (16) which are adapted to apply tensile forces (19) between the separated piece (17) of tubing and the residual tubing (18) of the advancing tubing (11) while separation takes place.

8. Plant (1) according to one of the above Claims,
**characterized in that**
the holder (10) has a mandrel (35), in particular a mandrel-shaped central electrode (36), and/or
a hose clamp (24) attached from the outside, and/or is fixed in the feeding direction (5) of the advancing tubing (11).

9. Plant (1) according to one of the above Claims,
**characterized in that**
the transfer means (3) has a passage channel (31) for the advancing tubing (11) and/or
can be moved forward and backward in the feeding direction (5) and/or
is adapted to move the passage channel (31) close to the holder (10), then to transfer the advancing tubing (11) at least with the piece (17) of tubing to be separated and, after separation of the piece (17) of tubing, to remove the residual tubing (18) of the advancing tubing (11) from the holder (10) through the passage channel.

10. Plant (1) according to one of Claims 7 through 9,
**characterized in that**
the tubing pulling means (16) has a retraction drive (21) and/or a clamp which is driven with respect to the tubing pulling means (15), preferably via a coupling to the transfer means (3), and/or
is adapted, in cooperation with the tubing traction holding means (15), to perform an elongation of 1% to 10% of the transition from the piece (17) of tubing to be separated to the residual tubing (18) between the tubing pulling means (16) and the tubing traction holding means (15), in particular from 2% to 6%, especially approximately 4%.

11. Plant (1) according to one of the above Claims,
**characterized in that**
the tubing transfer station (2) has a knife (42) which is driven, namely from a standby position (50) through the advancing tubing (11).

12. Plant (1) according to Claim 11,
**characterized in that**
the knife (42) is driven from the standby position (50) through the advancing tubing (11), the knife (42) being driven to release itself from the residual tubing (18) after separation, namely longitudinally in the tubing direction or with a motion component (53) longitudinally in the tubing direction.

13. Plant (1) according to Claim 11 or 12,
**characterized in that**
the knife (42) has a tilting drive (44).

14. Plant (1) according to Claim 13,
**characterized in that**
the tilting drive (44) is adapted to tilt the knife (42) away from the transfer means (3).

15. Plant (1) according to one of Claims 11 through 14,
**characterized in that**
the knife (42) is driven to move through the tubing (11) at an inclined angle, and/or
the knife (42) has a maximum thickness of 1 mm, in particular of 0.8 mm, preferably of 0.5 mm, particularly preferably of less than 0.2 mm.

16. Plant (1) according to one of Claims 11 through 15,
**characterized in that**
the tubing pulling means (16) and the knife (42) are arranged on the transfer means side whereas the tubing traction holding means (15) is arranged on the holder side.

17. Method of manufacturing a pouch for medical purposes, where a tubing advancing to a tubing transfer station (2) is transferred to a holder (10) for separation, at the holder, of a piece (11) of tubing transferred to the holder (10) from a residual tubing (18) of the advancing tubing (11) so that subsequently the piece (17) of tubing is connected to two sheets of film so as to form an access to the pouch; in particular using a plant (1) according to one of the above Claims,
**characterized in that**
during separation of the piece (17) of tubing from the residual tubing (18) of the advancing tubing (11), at least one air nozzle (55, 57) with a pressure difference means (59, 60) for creating an overpressure and/or a negative pressure at the air nozzle (55, 57) is operated, an effective range (58) of the at least one air nozzle (55, 57) being directed at the designated site (20) for separating and the pressure difference means (59, 60) being operated in such a manner during separation that an air flow forced by air which is blown out and/or air which is aspirated is adjusted at the designated site (20) for separation in order to remove tubing material residual particles from the designated site (20) or from the piece (17) of tubing, respectively, and also from the residual tubing (18) by means of the forced air flow (56) during separation of the piece (17) of tubing from the advancing tubing (11).

18. Method according to Claim 17,
**characterized in that**
air is blown out through a first air nozzle (55) and aspirated through another air nozzle (57).

19. Method according to Claim 17 or 18,
**characterized in that**
an air ionization unit for ionization of air to be fed to the at least one air nozzle is operated and ionized air is blown out through the at least one air nozzle (55, 57).

20. Method according to Claims 17 through 19,
**characterized in that**
the following steps are performed:
feeding the advancing tubing (11) to the holder (10);
tight retention of the advancing tubing (11) at the piece (17) of tubing to be separated, on the side of the holder (10) by means of a tubing traction holding means (15);
c. longitudinal pulling of the advancing tubing (11) between the tubing traction holding means (15) and a feeding system so that tensile forces act on the designated site (20) for separation; and
d. separating the piece (17) of tubing from the residual tubing (18) of the advancing tubing (11).

21. Method according to one of Claims 17 through 20,
**characterized in that**
feeding of the tubing (11) is performed through a passage channel (31)
and/or
feeding of the advancing tubing (11) is performed to a mandrel (35),
in particular to a central electrode (36).

22. Method according to one of Claims 17 through 21,
**characterized in that**
the advancing tubing (11) is fixed against retraction, in particular, clamped from the outside, with the holder (10) at a piece (17) of tubing to be separated.

23. Method according to one of Claims 17 through 22,
**characterized in that**
the holder (10) and the transfer means (3) are spaced from each other, in particular, the transfer means (3) is retracted, with the advancing tubing (11) being elongated at the designated site (20) for separation.

24. Method according to Claim 23,
**characterized in that**
separation takes place by means of a knife (42) which is guided through the advancing tubing (11) during the tensile force acting on the designated site (20) for separation so that the advancing tubing (11) follows the tensile force at least on one side, preferably on both sides, of the separated site and so that a gap (48) results after separation which is wider than a thickness of the knife (42).

25. Method according to Claim 24,
**characterized in that**
after separation, the knife (42) is released from the tubing (11) by means of a movement with one component along a tensile force direction; and/or
the knife (42) is tilted for release; and/or
the knife (42) is heated for separation to a temperature between 40°C and 70°C, in particular between 40°C and 60°C, especially about 50°C.

26. (not searched) Method according to one of Claims 17 through 25,
**characterized in that**
elongation of the tubing (11) is performed with a linear expansion between 1% and 10%, preferably from 2% to 6%, particularly preferably approximately 4%.

27. Method according to one of Claims 17 through 26,
**characterized in that**
separation takes place without the use of alcoholic lubricants.

## Revendications

1. Système (1) de fabrication d'un sac à des fins médicales comprenant un poste de transfert de flexible (2) avec un moyen de transfert (3) pour transférer un flexible (11) arrivant au poste de transfert de flexible (2) sur un support (10)
et pour la séparation localisée d'un bout de flexible (17) d'un reste de flexible (18) du flexible (11) arrivant transféré sur le support (10) pour pouvoir relier ensuite le bout de flexible séparé (17) à deux couches de film pour former un accès au sac,
**caractérisé en ce que**
le poste de transfert de flexible (2) comprend au moins une buse d'air (55, 57), sachant qu'une zone active (58) d'au moins une buse d'air (55, 57) est dirigée sur un emplacement désigné (20) pour la séparation de telle manière qu'un débit d'air (56) forcé par l'air soufflé et/ou l'air aspiré sur l'emplacement désigné (20) peut être réglé pour la séparation pour éliminer par le débit d'air forcé (56) les particules résiduelles de matériau de flexible produites lors de la séparation du bout de flexible (17) du flexible (11) arrivant de l'emplacement désigné (20) ou du bout de flexible (17) et également encore du reste de flexible (18).

2. Système (1) selon la revendication 1,
**caractérisé en ce qu'**
au moins une buse d'air (55, 57) comporte un moyen de différence de pression (59, 60) pour produire une surpression et/ou une dépression sur la buse d'air (55, 57) de telle manière que pendant le fonctionnement du moyen de différence de pression (59, 60) un débit d'air (56) forcé par l'air soufflé et/ou l'air aspiré peut être réglé à l'emplacement désigné (20) pour séparation.

3. Système (1) selon la revendication 1 ou 2,
**caractérisé en ce que**
deux buses d'air (55, 57) sont prévues, dont une première buse d'air (55) est agencée pour souffler de l'air et une deuxième buse d'air (57) pour aspirer de l'air.

4. Système (1) selon l'une quelconque des revendications précédentes,
**caractérisé en ce que**
l'emplacement désigné (20) pour la séparation est disposé entre les buses d'air (55, 57).

5. Système (1) selon l'une quelconque des revendications précédentes,
**caractérisé en ce que**
la deuxième buse d'air (57) pour aspirer de l'air est disposée pouvant pivoter sur le poste de transfert de flexible (2).

6. Système (1) selon l'une quelconque des revendications précédentes,
**caractérisé en ce que**
la deuxième buse d'air (57) pour aspirer de l'air est disposée sur un dispositif de maintien pivotable (41) pour maintenir un moyen de séparation (42) pour séparer le flexible (11) arrivant.

7. Système (1) selon l'une quelconque des revendications précédentes,
**caractérisé en ce que**
le poste de transfert de flexible (2) comporte côté support un moyen de retenue de flexible (15) et de l'autre côté un moyen d'étirage de flexible (16), lequel est agencé à cet effet pour mettre en œuvre des forces de traction (19) entre le bout de flexible séparé (17) et le reste de flexible (18) du flexible (11) arrivant pendant que la séparation a lieu.

8. Système (1) selon l'une quelconque des revendications précédentes,
**caractérisé en ce que**
le support (10) comporte une électrode à noyau (35), en particulier une électrode centrale (36) en forme de mandrin, et/ou
comporte un serrage de flexible (24) saisissant de l'extérieur, et/ou
est fixé en direction de mise en œuvre (5) du flexible (11) arrivant.

9. Système (1) selon l'une quelconque des revendications précédentes,
**caractérisé en ce que**
le moyen de transfert (3) comporte un conduit de passage (31) pour le flexible (11) arrivant, et/ou
peut être avancé et reculé dans la direction de mise en œuvre (5), et/ou
est agencé à cet effet pour déplacer le conduit de passage (31) près du support (10), puis transférer le flexible (11) arrivant au moins avec le bout de flexible à séparer (17) et éloigner du support (10) après séparation du bout de flexible (17) le reste de flexible (18) du flexible (11) arrivant à nouveau à travers le conduit de passage.

10. Système (1) selon l'une quelconque des revendications 7 à 9,
**caractérisé en ce que**
le moyen d'étirage de flexible (16) comporte un système de rappel (21) et/ou un serrage, lequel est entraîné par rapport au moyen de retenue de flexible (15), de préférence par un accouplement avec le moyen de transfert (3),
et/ou
est agencé à cet effet pour communiquer en conjonction avec le moyen de retenue de flexible (15) une expansion de 1 % à 10 % au transfert du bout de flexible à séparer (17) au reste de flexible (18) entre le moyen d'étirage de flexible (16) et le moyen de retenue de flexible (15), en particulier de 2 % à 6 %, surtout d'environ 4 %.

11. Système (1) selon l'une quelconque des revendications précédentes,
**caractérisé en ce que**
le poste de transfert de flexible (2) comporte un couteau (42), lequel est actionné en réalité d'une position d'attente (50) à travers le flexible (11) arrivant.

12. Système (1) selon la revendication 11,
**caractérisé en ce que**
le couteau (42) est actionné de la position d'attente (50) à travers le flexible (11) arrivant, sachant que le couteau (42) est actionné après la séparation pour se libérer du reste de flexible (18) et effectivement longitudinalement en direction du flexible ou avec un composant de mouvement (53) longitudinalement en direction du flexible.

13. Système (1) selon la revendication 11 ou 12,
**caractérisé en ce que**
le couteau (42) comporte un système de bascule (44).

14. Système (1) selon la revendication 13,
**caractérisé en ce que**
le système de bascule (44) est agencé de manière à faire basculer le couteau (42) en s'éloignant du moyen de transfert (3).

15. Système (1) selon l'une quelconque des revendications 11 à 14,
**caractérisé en ce que**
le couteau (42) est actionné se déplaçant de façon inclinée à travers le flexible (11), et/ou
le couteau (42) comporte une épaisseur de 1 mm maximum, surtout de 0,8 mm maximum, de préférence de 0,5 mm maximum, en particulier de préférence inférieure à 0,2 mm.

16. Système (1) selon l'une quelconque des revendications 11 à 15,
**caractérisé en ce que**
le moyen d'airage de flexible (16) et le couteau (42) sont disposés du côté du moyen de transfert, le moyen de retenue de flexible (15) étant par contre disposé du côté du support.

17. Procédé de fabrication d'un sac à des fins médicales pour lequel un flexible arrivant vers un poste de transfert de flexible (2) est transféré au moyen d'un moyen de transfert (3) sur un support (10) pour la séparation locale d'un bout de flexible (11) transféré sur le support (10) d'un reste de flexible (18) du flexible (11) arrivant de telle manière que le bout de flexible (17) est ensuite relié à deux couches de film pour former un accès au sac, en particulier en utilisant un système (1) selon l'une quelconque des revendications précédentes,
**caractérisé en ce que**
pendant la séparation du bout de flexible (17) du reste de flexible (18) du flexible (11) arrivant au moins une buse d'air (55, 57) est activée avec un moyen de différence de pression (59, 60) pour produire une surpression et/ou une dépression sur la buse d'air (55, 57), sachant qu'une zone active (58) d'au moins une buse d'air (55, 57) est dirigée sur l'emplacement désigné (20) pour la séparation et sachant que le moyen de différence de pression (59, 60) est activé pendant la séparation de telle manière qu'un débit d'air forcé par de l'air soufflé et/ou de l'air aspiré est réglé sur l'emplacement désigné (20) pour la séparation pour éliminer par le débit d'air forcé (56) les particules résiduelles de matériau de flexible produites lors de la séparation du bout de flexible (17) du flexible (11) arrivant de l'emplacement désigné (20) ou du bout de flexible (17) et également encore du reste de flexible (18).

18. Procédé selon la revendication 17,
**caractérisé en ce que**
de l'air est soufflé à travers une première buse d'air (55) et de l'air est aspiré à travers une autre buse d'air (57)

19. Procédé selon la revendication 17 ou 18,
**caractérisé en ce qu'**
un système d'ionisation de l'air est activé pour ioniser de l'air arrivant d'au moins une buse d'air et de l'air ionisé est soufflé à travers au moins une buse d'air (55, 57).

20. Procédé selon la revendication 17 à 19,
**caractérisé en ce que**
les étapes suivantes sont exécutées:
alimentation du flexible (11) arrivant vers le support (10), maintien ferme du flexible (11) arrivant sur le bout de flexible à séparer (17) sur les côtés du support (10) au moyen d'un moyen de retenue de flexible (15),
c. étirage longitudinal du flexible (11) arrivant entre le moyen de retenue de flexible (15) et un système d'alimentation de telle manière que des forces de traction agissent sur l'emplacement désigné (20) pour la séparation, et
d. séparation du bout de flexible (17) du reste de flexible (18) du flexible (11) arrivant.

21. Procédé selon l'une quelconque des revendications 17 à 20,
**caractérisé en ce que**
alimentation du flexible (11) a lieu à travers un conduit de passage (31)
et/ou
alimentation du flexible (11) arrivant a lieu sur une électrode à noyau (35), en particulier sur une électrode centrale (36).

22. Procédé selon l'une quelconque des revendications 17 à 21,
**caractérisé en ce que**
le flexible (11) arrivant est fixé contre tout recul en arrière avec le support (10) sur un bout de flexible à séparer (17) et est surtout fixé fermement de l'extérieur.

23. Procédé selon l'une quelconque des revendications 17 à 22,
**caractérisé en ce que**
le support (10) et le moyen de transfert (3) sont éloignés l'un de l'autre, le moyen de transfert (3) est en particulier tiré en arrière et le flexible (11) arrivant est tiré à cet effet à l'emplacement désigné (20) pour la séparation à la mise à longueur.

24. Procédé selon la revendication 23,
**caractérisé en ce que**
la séparation a lieu au moyen d'un couteau (42), lequel est guidé par le flexible (11) arrivant pendant la force de traction agissant sur l'emplacement désigné (20) pour la séparation de telle manière que le flexible (11) arrivant suit la force de traction au moins d'un côté, de préférence des deux côtés de l'emplacement séparé et qu'il en résulte une faille (48) après la séparation, qui est plus large qu'une épaisseur du couteau (42).

25. Procédé selon la revendication 24,
**caractérisé en ce que**
le couteau (42) est libéré du flexible (11) après la séparation et comporte effectivement au moyen d'un mouvement un composant le long d'une direction de force de traction, et/ou
le couteau (42) est basculé pour libération, et/ou le couteau (42) est chauffé pour séparation à une température se situant entre 40°C et 70°C, surtout entre 40°C et 60°C, en particulier autour de 50°C.

26. (non recherché) Procédé selon l'une quelconque des revendications 17 à 25,
**caractérisé en ce que**
l'étirage longitudinal du flexible (11) est exécuté avec une expansion longitudinale se situant entre 1% et 10%, de préférence de 2 % à 6 %, en particulier de préférence environ 4 %.

27. Procédé selon l'une quelconque des revendications 17 à 26,
**caractérisé en ce que**
la séparation a lieu en évitant un lubrifiant alcoolique.
